Europäisches Patentamt

European Patent Office

Office européen des brevets

(11) Veröffentlichungsnummer: **0 478 842 A1**

# (12) EUROPÄISCHE PATENTANMELDUNG

(21) Anmeldenummer: **90119187.4**

(22) Anmeldetag: **05.10.90**

(51) Int. Cl.⁵: **B01D 69/00**, A61M 1/36

(43) Veröffentlichungstag der Anmeldung:
**08.04.92 Patentblatt 92/15**

(84) Benannte Vertragsstaaten:
**AT BE CH DE DK ES FR GB GR IT LI LU NL SE**

(71) Anmelder: **PALL BIOMEDIZIN GmbH**
**Philip-Reis-Strasse 6**
**W-6072 Dreieich 6(DE)**

(72) Erfinder: **Wollinsky, Kurt H., Dr.**
**Schelmenweg 11**
**W-7901 Illerkirchberg(DE)**
Erfinder: **Saefkow, Michael W. K., Dr.**
**Finkenweg 6**
**W-6070 Langen(DE)**

(74) Vertreter: **Dost, Wolfgang,**
**Dr.rer.nat.,Dipl.-Chem. et al**
**Patent- & Rechtsanwälte Bardehle .**
**Pagenberg . Dost . Altenburg . Frohwitter .**
**Geissler & Partner Galileiplatz 1 Postfach 86**
**06 20**
**W-8000 München 86(DE)**

(54) **Filter zur Filtration menschlicher Cerebronspinalflüssigkeit.**

(57) Ein Filter zur Anwendung in einem Verfahren zur Filtration von Liquor cerebrospinalis enthält eine Membranfilterschicht mit einer Porenweite von 0,04 - 0,45 $\mu$m und einer Schichtdicke von 0,1 - 1 mm, wobei das Filter eine geometrische Oberfläche von 50 - 300 cm$^2$ und eine Pyrogen-Abscheideleistung von mindestens 500 $\mu$g besitzt.

EP 0 478 842 A1

Die Erfindung betrifft ein Filter zur Filtration von Liquor cerebrospinalis, insbesondere zur Behandlung von neurologischen Krankheitsbildern, die mit entzündlichen Prozessen (Neuritiden, z. B. Polyradikulitis, Radikuloneuritis oder Guillain-Barré-Syndrom (GBS) einhergehen oder nicht entzündliche Prozesse (Neuropathien) darstellen, sowie Intoxikationen des Liquors (z. B. Diphterie, Tetanus) oder Infektionen durch Bakterien (z. B. Borreliose) oder Viren (z. B. Virus-Enzephalitis, Polio), die sich am Nervensystem ursächlich oder symptomatisch manifestieren.

Bei GBS z. B. handelt es sich um eine aufsteigende motorische und sensible Lähmung bzw. aufsteigende Nervenlähmung, einschließlich der unteren Hirnnerven, die zunächst die Extremitäten erfaßt, so daß der Patient gehunfähig bzw. bettlägerig wird, und bis zur vollständigen Lähmung aller Muskeln führen kann, so daß eine Langzeitbeatmung des Patienten erforderlich wird. Eine Degeneration von Nerven mit Langzeitschäden ist möglich.

Die vorgenannten Krankheitsbilder schlagen sich u.a. in einer Veränderung des Liquorbefundes nieder, jedoch steht eine Klärung der Ursachen der Krankheitsbilder bisher aus, und insbesondere konnte auch eine Kausalität zwischen den veränderten Liquorbefunden und den Krankheitsbildern nicht hergestellt werden. Die Therapie des Guillain-Barré-Syndroms besteht in der therapeutischen Plasmapherese, d.h. einem (partiellen) Plasmaaustausch, wobei nach Blutentnahme vom Patienten apparative Trennung von korpuskulären Elementen und Plasma, z.B. mittels Zentrifuge erfolgt. Die korpuskulären Bestandteile werden nach vollständigem oder partiellen Volumenersatz des entzogenen Plasmas in Plasmaersatz resuspendiert und dem Patienten reinfundiert. Bei zahlreichen Patienten ist jedoch mit der Methode der Plasmapherese nur eine geringe oder überhaupt keine Besserung des Krankheitsbildes zu erreichen.

Es wurde nun gefunden, daß man auch in den zuletzt genannten Fällen eine erhebliche Verbesserung des Krankheitsbildes bis hin zur Beschwerdefreiheit erreichen kann, wenn man den Liquor dieser Patienten einer Filtration durch ein besonderes Filter unterwirft.

Gegenstand der Erfindung ist somit ein Filter zur Anwendung in einem Verfahren zur Filtration von Liquor cerebrospinalis, das gekennzeichnet ist durch eine Membranfilterschicht mit einer Porenweite von 0,04-0,45$\mu$m und einer Schichtdicke von 0,1-1 mm, wobei das Filter eine geometrische Oberfläche von 50-300 cm$^2$ und eine Pyrogen-Abscheideleistung von mindestens 500 $\mu$g besitzt.

Die Pyrogen-Abscheideleistung, angegeben in $\mu$g, bedeutet, daß das Filter die genannte Menge Pyrogen, bezogen auf ein Standard-E. coli-Endotoxin im Konzentrationsbereich zwischen 0,6 und 6,9 ng/ml im Ausgangsmaterial zurückhält, wobei die Konzentration im Filtrat unterhalb der Schwelle des LAL-Tests (etwa 0,006 ng/ml) liegt.

Pyrogene (fiebererzeugende Stoffe) sind in erster Linie hitzebeständige, dialysierbare Stoffe von apathogenen und pathogenen Bakterien, Pilzen oder Viren, die parenteral beim Menschen in kleinsten Mengen ( <1 $\mu$g/kg) Schüttelfrost und Temperaturanstieg (Fieber) bewirken. Ihrem chemischen Charakter nach handelt es sich bei den Pyrogenen hauptsächlich um Oligo-, Poly- und Lipopolysaccharide oder Polypeptide, wobei die am stärksten wirksamen Pyrogenen diejenigen gramnegativer Bakterien sind.

Von Bedeutung sind die Pyrogene vor allem als Verunreinigungen in Injektions- bzw. Infusionslösungen, aus denen sie mittels sog. Bakterienfilter entfernt werden müssen. Definitionsgemäß handelt es sich bei Bakterienfiltern um mikroporöses Material, mit dessen Hilfe Bakterien durch Sieb- oder Adsorptionswirkung aus Gasen oder Flüssigkeiten zurückgehalten werden können, z.B. Ganzglas-, Membran- oder Siebfilter (vergl. Roche Lexikon Medizin, 1. Aufl. 1984, Seite 135). Die Bestimmung der Pyrogen-Abscheideleistung erfolgt mit einem Standardpyrogen, nämlich mit einem E. coli-Endotoxin, wobei die Wirksamkeit der Abscheidung mit dem Kaninchentest oder dem LAL-Test (Limulus-Amöbozyten-Lysat-Test) überprüft wird (vergl. Pharm. Ind. 47 (1985) 407-411). Handelsübliche Bakterienfilter, die als Infusionsfilter zum Schutz des Patienten Verwendung finden, besitzen Pyrogen-Abscheideleistungen unter 100 $\mu$g, wobei die geometrische Filterfläche etwa 10 cm$^2$ beträgt.

Die Anwendung des erfindungsgemäßen Filters in dem Verfahren zur Filtration des Liquor cerebrospinalis kann in höchst einfacher Weise erfolgen. In der Praxis hat sich hierzu eine Verfahrensweise bewährt, bei der nach Plazierung eines intrathekalen Katheters mit zwei Dreiwegehähnen, einer 10 ml Spritze und dem Liquorfilter ein geschlossenes System installiert wird. Manuell werden dann jeweils 10-40 ml Liquor entnommen und nach Filterpassage zurückgegeben. Mit dieser Methode wurden 6 GBS-Patienten behandelt, 4 mit einer akuten 2 mit einer chronischen Verlaufsform, bei 3 Patienten war zuvor eine intensive Plasmapherese ohne Wirkung geblieben. Es wurde an bis zu 5 Tagen in 1-2 täglichen Sitzungen jeweils maximal 150 ml Liquor filtriert. Das Verfahren wurde von allen Patienten gut und ohne Komplikationen toleriert. Kopfschmerzen traten nur flüchtig und nicht in der sonst nach Lumbalpunktionen beobachteten Stärke auf. Bei allen Patienten konnte in zeitlichem Zusammenhang mit der Liquorfiltration, manchmal noch während dieser Behandlung, eine unterschiedlich deutliche, teilweise erhebliche Besserung des klinischen Befundes festgestellt werden, bei den akuten und frühbehandelten Patienten deutlicher und schneller als bei den chronischen.

Eine Filtration des Liquors kann auch bei anderen Krankheitsbildern angezeigt sein, die mit einer Veränderung des Liquors einhergehen, z. B. bei multipler Sklerose oder ALS.

Bei dem erfindungsgemäßen Filter besitzt die Membranfilterschicht eine Porengröße von 0,04-0,45 $\mu$m, vorzugsweise 0,1-0,3 $\mu$m und insbesondere von 0,1-0,2 $\mu$m. Membranfilterschichten mit derartiger Porenweite sind an sich bekannt und finden in Bakterienfiltern für die Verabreichung von Infusionslösungen Verwendung, wobei Membranen mit Porenweiten unterhalb von 0,1 $\mu$m auch als Virenfilter angewendet werden. Die üblichen Bakterienfilter sind jedoch erfindungsgemäß ungeeignet, da bei deren Anwendung für die Liquorfiltration keine signifikante Veränderung erzielt wird. Aufgrund der ungeklärten Verhältnisse beim Guillain-Barré-Syndrom war auch gar nicht zu erwarten, daß man mit dem beanspruchten Filter eine signifikante Verbesserung des Krankheitsbildes würde erreichen können.

Das erfindungsgemäße Filter besitzt eine Pyrogen-Abscheideleistung von mindestens 500 $\mu$g, z.B. mindestens 600 oder 700 $\mu$g, und vorzugsweise von mindestens 1000 $\mu$g, wobei ein Bereich von 1000-2000 $\mu$g besonders bevorzugt ist.

Die Membranfilterschicht besitzt eine Schichtdicke von 0,1-1 mm, wobei das Filter eine geometrische Oberfläche von 50-300 cm$^2$ aufweist.

Innerhalb der genannten Bereiche von 0,04-0,45 $\mu$m für die Porenweite, 50-300 cm$^2$ für die geometrische Oberfläche und 0,1-1 mm für die Dicke der Membranfilterschicht erfolgt eine Abstimmung derart, daß die geforderte Pyrogen-Abscheideleistung von mindestens 500 $\mu$g gewährleistet ist und für die praktische Anwendung eine ausreichende Fluxrate zur Verfügung steht. Im allgemeinen ist die Fluxrate etwa proportional zur Porenweite und zur Oberfläche und umgekehrt etwa proportional zur Schichtdicke, während die Abscheideleistung proportional zur Schichtdicke und zur geometrischen Oberfläche ist.

Ein weiterer Parameter zur Steuerung der Pyrogen-Abscheideleistung und der Fluxrate ist das Porenvolumen, ausgedrückt als Prozentsatz des Porenvolumens, bezogen auf das Gesamtvolumen, wobei innerhalb gewisser Grenzen sowohl die Abscheideleistung als auch die Fluxrate dem Porenvolumen etwa proportional sind. Das Porenvolumen sollte so groß wie möglich sein, um das Filter möglichst klein halten zu können. Dem Porenvolumen sind jedoch nach oben Grenzen gesetzt. In der Praxis beträgt das Porenvolumen im allgemeinen 50-90%, wobei aus dem vorgenannten Grund die höheren Werte bevorzugt sind.

Als Material für die Membranfilterschicht kommen alle inerten polymeren Werkstoffe in Frage, aus denen sich nach den bekannten Verfahren Membranen mit den angegeben Porenbereichen herstellen lassen. Beispiele für geeignete Werkstoffe sind gegenüber Körperflüssigkeiten inerte Kunststoffe, wie Polyolefine, z. B. Polyethylen oder Polypropylen, Polyamide, z. B. Nylon 6,6 oder Polycaprolactam, Polyester oder Polyvinylidenfluorid.

In einer bevorzugten Ausführungsform handelt es sich bei dem Kunststoffmaterial für die Membranfilterschicht um ein ladungsmodifiziertes Polymeres, weil sich hiermit die Abscheideleistung leichter erreichen läßt. Ladungsmodifizierte Polymere, und zwar sowohl mit positivem Zetapotential als auch mit negativem Zetapotential, sind in der US-PS 4 702 840 und der EP-PA 0 087 228 beschrieben, auf die hier vollinhaltlich Bezug genommen wird.

Die in der US-PS 4 702 840 beschriebenen ladungsmodifizierten Polymeren mit positivem Zetapotential werden auch als kationische Polymere bezeichnet. Geeignet ist z. B. ein Polyamid, z. B. Nylon-6,6, dessen Oberfläche mit einem quartäre Amoniumgruppen tragenden, hitzehärtbaren Polymeren modifiziert worden ist, wobei solche kationischen Polymeren bevorzugt sind, die über funktionelle Epoxidgruppen eine Vernetzung bewirken, vergleiche hierzu Spalte 6 der US-PS 4 702 840. Da die positive Ladung dieser Polymeren von der Anwesenheit der quartären Amoniumgruppen herrührt, behalten sie ihre positive Ladung in saurem, neutralem und alkalischem pH-Bereich.

Bei den ladungsmodifizierten Polymeren der EP-PA 0 087 228 handelt es sich um solche mit negativem Zetapotential, die auch als anionische Polymere bezeichnet werden. Als Quelle für die negative Ladung dienen ionisierbare funktionelle polare Gruppen, wie Carboxylgruppen, Sulfonsäuregruppen, phenolische Aminogruppen, Sulfhydryl-, Sulfid-, Thiocarbonyl-, Phosphin-, Phosphoryl- oder Thiophosphorylgruppen (Seiten 15/16 der EP-PA 0 087 228). Carboxylgruppen sind bevorzugt.

In den beiden vorgenannten Patentveröffentlichungen ist auch die Technik der Herstellung von Membranfilterschichten mit geeigneten Porenweiten und Porenvolumina beschrieben.

In einer weiteren bevorzugten Ausführungsform ist das erfindungsgemäße Filter als Verbundfilter ausgebildet, wobei das Filter eine zweite Membranfilterschicht mit einer Porenweite im Bereich von 0,1-5 $\mu$m und einer Schichtdicke von 0,1-1 mm enthält. Für diesen Fall kann die erste Membranfilterschicht sehr dünn gehalten werden, da deren Funktion lediglich noch in der Bakterien- bzw. Virenabscheidung besteht, während in der zweiten (vorgeschalteten) Schicht durch Adsorption bereits die Hauptmenge der störenden Bestandteile erfolgt ist. In dieser Ausführungsform wird somit der ganz wesentliche Teil der Abscheideleistung von der vorgeschalteten zweiten Schicht erbracht. Bei der zweiten Filterschicht kommt es in erster Linie auf die Adsorptionskapazität und weniger auf die Porenweite an, weshalb hier auch größere Porenweiten, z. B. von 0,8 $\mu$m oder 1,2 $\mu$m oder bis zu 5 $\mu$m geeignet sind. Auch bezüglich der zweiten Schicht werden jedoch mit kleineren Porenweiten die besseren Ergebnisse erzielt, weshalb Porenweiten von 0,1-0,45 $\mu$m bevorzugt sind.

Die Schichtdicke der zweiten Filterschicht beträgt 0,1-1 mm. Mit zunehmender Porenweite (und mit zunehmendem Porenvolumen) kann infolge der größeren Fluxrate die Schichtdicke vergrößert werden, was andererseits eine Änderung der geometrischen Oberfläche des Filters im Bereich von 50-300 cm$^2$ zu kleineren Werten hin bei Aufrechterhaltung der Mindest-Pyrogen-Abscheideleistung von 500 $\mu$g ermöglicht.

Das erfindungsgemäße Filter ist somit einschichtig oder mehrschichtig ausgebildet. Bei der einschichtigen Ausführungsform besteht die einzige Membranfilterschicht vorzugsweise aus einem ladungsmodifizierten Polymeren, wie vorstehend beschrieben.

Bei der mehrschichtigen Ausführungsform besteht das Verbundfilter aus mindestens zwei Schichten, wobei vorzugsweise die zweite (vorgeschaltete) Filterschicht aus einem ladungsmodifizierten Polymeren besteht. In einer speziellen Ausführungsform dieses Verbundfilters besteht die erste Filterschicht aus einem ladungsneutralen Polymeren, z. B. Nylon 6,6, wobei die Ladung sowohl positiv als auch negativ sein kann (positives oder negatives Zetapotential). In einer weiteren speziellen Ausführungsform eines zweischichtigen Filters ist die erste Schicht aus einem positiv ladungsmodifizierten Material und die zweite (vorgeschaltete) Schicht aus einem negativ ladungsmodifizierten Material. In einer weiteren speziellen Ausführungsform eines dreischichtigen Filters besteht die zweite Schicht aus einem positiv ladungsmodifizierten Material und die dritte Schicht aus einem negativ ladungsmodifizierten Material.

Für die Anwendung in der Praxis ist das erfindungsgemäße Filter, unabhängig von Anzahl und Art der Schichten, mit einer oder mehreren Stütz- oder Trägerschichten ausgerüstet, die dem Filter mechanische Festigkeit verleihen, ohne die Gebrauchseigenschaften zu verändern. Weiterhin ist das Filter für die praktische Anwendung vorzugsweise in einem Filtergehäuse mit entsprechenden Anschlüssen angeordnet, wobei die vorgenannten Stützschichten auch in das Filtergehäuse integriert sein können.

In einer weiteren bevorzugten Ausführungsform ist das erfindungsgemäße Filter mit einer Entlüftungseinrichtung ausgerüstet. Es hat sich nämlich gezeigt, daß es während des Betriebes des Liquorfilters zur Entwicklung von Gasblasen kommen kann, die ihrerseits zu einer Blockierung des Filters führen können. Die Entlüftungseinrichtung ermöglicht den Austritt dieser Gasblasen aus dem Filtergehäuse unter Aufrechterhaltung der Sterilität. Als Entlüftungseinrichtung dient z.B. ein übliches Entlüftungsventil. Vorzugsweise ist für diesen Zweck jedoch eine Entlüftungsmembran vorgesehen, bei der es sich um eine hydrophobe Membran mit derartiger Porenweite handelt, die einerseits den Austritt von Liquorflüssigkeit verhindert, anderseits jedoch den Durchtritt der Gasblasen beim Filtrationsdruck ermöglicht. Die Anordnung der Entlüftungsmembran erfolgt auf der unreinen Seite des Filtergehäuses, d. h. auf derjenigen Seite, auf der der zu filtrierende Liquor in das Filtergehäuse eintritt.

Im folgenden wird die Erfindung anhand der Zeichnungen erläutert, wobei die Figuren 1-4 in schematischer Darstellung einschichtige und mehrschichtige Ausführungsformen von erfindungsgemäßen Liquorfiltern wie folgt zeigen:

- Figur 1 eine einschichtige Membranfilterschicht für ein erfindungsgemäßes Liquorfilter,
- Figur 2 eine zweischichtige Ausführungsform,
- Figur 3 ebenfalls eine zweischichtige Ausführungsform, und
- Figur 4 eine dreischichtige Ausführungsform.

Dargestellt sind jeweils nur die einzelnen Schichten, ohne Stützschichten und Gehäuse.

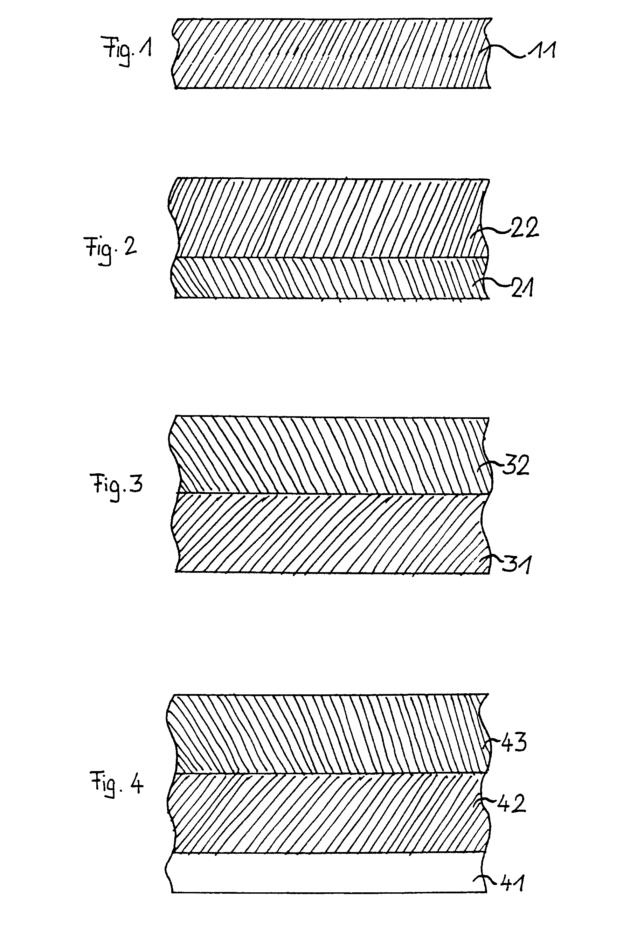

Figur 1 zeigt eine Membranfilterschicht 11 mit einer Schichtdicke von 0,2 mm und einer Porenweite von 0,2 $\mu$m. Das Material besteht aus Nylon-6,6, das durch Einführung von quartären Amoniumgruppen positiv ladungsmodifiziert ist, d. h. ein positives Zetapotential aufweist. Ähnliche Filterschichten sind von der Firma Pall unter der Bezeichnung Posidyne im Handel erhältlich.

Ein mit dieser Membranfilterschicht hergestelltes Liquorfilter besitzt bei einer geometrischen Oberfläche des Filters von 160 cm$^2$ eine Pyrogen-Abscheideleistung von 560 $\mu$g.

Figur 2 zeigt eine zweischichtige Ausführungsform. Die erste Schicht 21 besteht aus Nylon-6,6, und besitzt eine Dicke von 0,1 mm sowie eine Porenweite von 0,04 $\mu$m.

Dieser ersten Schicht 21 ist eine zweite Schicht 22 aus dem Posidyne-Material von Beispiel 1, Schichtdicke 0,2 mm Porenweite 0,2 $\mu$m, vorgeschaltet.

Bei einer geometrischen Oberfläche von 160 cm besitzt ein hieraus hergestelltes Filter eine Pyrogen-Abscheideleistung von 560 $\mu$g. Aufgrund der Porenweite von 0,04$\mu$m für die erste Schicht 21 ist das Verbundfilter Viren-retentiv.

In Figur 3 besteht die erste Membranfilterschicht 31 aus Posidyne mit einer Dicke von 0,2 mm und einer Porenweite von 0,2 $\mu$m. Die zweite Schicht 32 besteht aus einem negativ ladungsmodifizierten Polymeren, d. h. einem Polymeren mit negativem Zetapotential. Membranen aus derartigem Material sind von der Firma Pall unter der Handelsbezeichnung Carboxidyne im Handel erhältlich. Die zweite Schicht 32 besitzt eine Dicke von 0,2 mm und eine Porenweite von 0,2 $\mu$m.

Ein aus diesem Verbundmaterial hergestelltes Liquorfilter besitzt bei einer geometrischen Oberfläche von 160 cm$^2$ eine Pyrogen-Abscheideleistung von 560 $\mu$g.

In Figur 4 besitzt die erste Schicht 41 eine Dicke von 0,1 mm, eine Porenweite von 0,04 $\mu$m und besteht aus Nylon-6,6. Diese erste Schicht 41 sorgt, wie bei dem Verbundfilter von Figur 2, für die Viren-Retention.

Die zweite Schicht 42 besteht aus Posidyne und besitzt eine Schichtdicke von 0,2 mm und eine Porenweite von 0,2 mm.

Die dritte Schicht 43 besitzt eine Dicke von 0,2 mm, eine Porenweite von 0,04 $\mu$m und besteht aus Carboxidyne.

Ein aus diesem Verbundmaterial hergestelltes Liquorfilter besitzt bei einer geometrischen Oberfläche von 160 cm$^2$ eine Pyrogen-Abscheideleistung von 560 $\mu$g.

**Patentansprüche**

1. Filter zur Anwendung in einem Verfahren zur Filtration von Liquor cerebrospinalis, gekennzeichnet durch eine Membranfilterschicht mit einer Porenweite von 0,04-0,45 $\mu$m und einer Schichtdicke von 0,1-1 mm, wobei das Filter eine geometrische Oberfläche von 50-300 cm$^2$ und eine Pyrogen-Abscheideleistung von mindestens 500 $\mu$g besitzt.

2. Filter nach Anspruch 1, dadurch gekennzeichnet, daß die Membranfilterschicht eine Porenweite von 0,1-0,3 $\mu$m besitzt.

3. Filter nach Anspruch 2, dadurch gekennzeichnet, daß die Membranfilterschicht eine Porenweite von 0,1-0,2 $\mu$m besitzt.

4. Filter nach einem der Ansprüche 1-3, dadurch gekennzeichnet, daß die Membranfilterschicht eine Pyrogen-Abscheideleistung von 1000-2000 $\mu$g besitzt.

5. Filter nach einem der Ansprüche 1-4, dadurch gekennzeichnet, daß die Menbranfilterschicht aus einem Polyamid besteht.

6. Filter nach einem der Ansprüche 1-4, dadurch gekennzeichnet, daß die Membranfilterschicht aus einem ladungsmodifizierten Polymeren besteht.

7. Filter nach Anspruch 1, dadurch gekennzeichnet, daß es als Verbundfilter ausgebildet ist, wobei der Membranfilterschicht eine zweite Membranfilterschicht mit einer Porenweite im Bereich von 0,1-5 $\mu$m und einer Schichtdicke von 0,1-1 mm vorgeschaltet ist.

8. Filter nach Anspruch 7, dadurch gekennzeichnet, daß die zweite Membranfilterschicht eine Porenweite von 0,1-0,45$\mu$m besitzt.

9. Filter nach Anspruch 7 oder 8, dadurch gekennzeichnet, daß die zweite Membranfilterschicht (22, 32, 42) aus einem ladungsmodifizierten Polymeren mit posititvem Zetapotential besteht.

10. Filter nach Anspruch 7 oder 8, dadurch gekennzeichnet, daß die erste Membranfilterschicht (31) aus einem ladungsmodifizierten Polymeren mit positivem Zetapotential und die zweite Membranfilterschicht (32) aus einem ladungsmodifizierten Polymeren mit negativem Zetapotential besteht.

11. Filter nach Anspruch 7 oder 8, dadurch gekennzeichnet, daß die zweite Membranfilterschicht 42 aus einem ladungsmodifizierten Polymeren mit positivem Zetapotential und eine dritte Membranfilterschicht 43 aus einem ladungsmodifizierten Polymeren mit negativem Zetapotential besteht.

12. Filter nach Anspruch 9 oder 11 dadurch gekennzeichnet, daß die erste Membranfilterschicht 21, 41 zur Virenretention eine Porenweite unterhalb von 0,1 $\mu$m, vorzugsweise 0,04 $\mu$m besitzt.

13. Filter nach einem der Ansprüche 1 bis 12, gekennzeichnet durch die Anwesenheit einer oder mehrerer Stützschichten, die dem Filter mechanische Festigkeit verleihen ohne die Filtereigenschaften zu verändern.

14. Filter nach einem der Ansprüche 1 bis 13, gekennzeichnet durch die Anwesenheit eines Filtergehäuses mit Anschlußstutzen zur Aufnahme des Filters.

15. Filter nach Anspruch 14, gekennzeichnet durch die Anwesenheit einer Entlüftungsmembran auf der unreinen Seite des Filtergehäuses.

Fig. 1

11

Fig. 2

22

21

Fig. 3

32

31

Fig. 4

43

42

41

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | MOLECULAR AND CELLULAR PROBES, vol. 1, no. 4, December 1987, pages 347-358; H.A. ROTBART et al.: "RNA target loss during solid phase hybridization of body fluids - a quantitative study" <br> – – – | | B 01 D 69/00 <br> A 61 M 1/36 |
| A | CHEMICAL ABSTRACTS, vol. 82, no. 17, 28th April 1975, page 116, abstract no. 107252d, Columbus, Ohio, US; K. MOCHIDA et al.: "Pyrogenic factor appearing in the cerebro-spinal fluid of febrile rabbits", & NIPPON YAKURIGAKU ZASSHI 1974, 70(3), 359-63 <br> – – – | | |
| A | BIOLOGICAL ABSTRACTS, vol. 84, no. 2, 1987, abstract no. 16637, Biological Abstracts Inc., Philadelphia, PA, US; L. PAGANO et al.: "Spinal fluid procoagulant activity in leuke-mic patients treated with intrathecal methotrexate", & HAE-MATOLOGIA 20(1): 41-48. 1987 <br> – – – | | |
| A | BIOLOGICAL ABSTRACTS, vol. 83, no. 5, 1987, abstract no. 46268, Biological Abstracts Inc., Philadelphia, PA, US; L. PAGANO et al.: "Cerbrospinal fluid procoagulant activity in patients affected by myclo-lymphoproliferative diseases", & ITAL. J. MED. 2(1): 7-9. 1986 <br> – – – | | |
| A | US-A-3 591 493 (R.A. ZEINEH) <br> – – – | | |
| A | EP-A-0 080 680 (CORDIS EUROPA N.V.) <br> – – – | | |
| A | EP-A-0 098 392 (GAMBRO DIALYSATOREN KG) <br> – – – – – | | |

**RECHERCHIERTE SACHGEBIETE (Int. Cl.5)**

B 01 D 69/00
A 61 M 1/00

Der vorliegende Recherchenbericht wurde für alle Patentansprüche erstellt

| Recherchenort | Abschlußdatum der Recherche | Prüfer |
|---|---|---|
| Den Haag | 05 Juni 91 | DEVISME F.R. |